# EUROPEAN PATENT SPECIFICATION

(11) **EP 3 608 651 B1**
(45) Date of publication and mention of the grant of the patent: **03.04.2024**
(21) Application number: 18870716.0
(22) Date of filing: 25.07.2018
(51) Int. Cl.: G01N 1/28, G01N 33/48, B01L 9/00

(54) **CENTRIFUGAL SMEARING SAMPLE CONTAINER HOLDER**
ZENTRIFUGALER BEHÄLTERHALTER FÜR ABSTRICHPROBE
SUPPORT DE RÉCIPIENT D'ÉCHANTILLON DE FROTTIS CENTRIFUGE

(30) Priority: 26.10.2017 JP 2017207338
(43) Date of publication of application: 12.02.2020
(73) Proprietor: Sakura Seiki Co., Ltd, Chikuma-shi, Nagano 387-0015 (JP); Sakura Finetek Japan Co., Ltd., Tokyo 103-0023 (JP)
(72) Inventor: TOYA, Matsumi, Chikuma-shi Nagano 387-0015 (JP)
(74) Representative: Mewburn Ellis LLP
(86) International application number: PCT/JP2018/027819
(87) International publication number: WO 2019/082461

(56) References cited:
- WO-A1-2014/167925
- JP-U- H0 291 944
- JP-U- S5 888 143
- JP-U- S6 283 951
- JP-U- S62 102 145
- US-A1- 2011 064 629

## Description

### Technical Field

The present invention relates to a centrifugal smearing sample container holder.

### Background Art

Cytological diagnoses have been widely used for the early detection of cancer, the medical examination, and the like because the invasiveness to a patient when a sample is collected is low, and a determination as to whether benign or malignant can be diagnosed for a short time. Examples of a liquid sample used in the cytological diagnosis include urine, a body-cavity fluid, and a puncture sucking washing liquid, and it is important to efficiently collect a small number of cells without degeneration. Centrifugal smearing devices can efficiently collect and smear cells for a short time using a centrifugal force, and are thus used in many pathology laboratories, research laboratories, and the like.

Conventionally, has been known a centrifugal smearing device in which a centrifugal smearing sample container holder is mounted thereto, and is rotated in a circumferential direction, thereby smearing cells by the centrifugal force on slide glass (PTL 1: JP-A-11-044621, PTL 2: JP-T-2006-513413, NPL 1: "Thermo Scientific Shandon Thin-layer Cell Preparation System", the Internet <URL:http://www.med.osaka-cu.ac.jp/Central-lab/16F/kikisitu/cytospin catalog.pdf>). Moreover, conventionally, a procedure in which a container containing a liquid sample, a packing, and slide glass are attached to a centrifugal smearing sample container holder has been known (NPL 2: "Thermo Scientific Shandon Cytoclip Slide Clip, TPX Sample Chambers and Filter Cards Instructions for Use", the Internet <URL:http://tools.thermofisher.com/content/sfs/manuals/D21681~.pdf>).
JP S62 83951 U describes a cell holder for centrifugal smearing. In the cell holder, a slide glass 8, a liquid permeable packing plate 7 having a hole 10 and a substrate 2 of a cell are stacked and attached to a centrifugal separator. The cell holder for centrifugal smearing is characterized in that a Oritate edge 22a is formed on four peripheries of a short plate 11 of the cell holder to surround a slide glass 8 or the like, and a reservoir part 2530 for a liquid separated from a liquid to be treated is provided in front of or behind the rectangular plate 11.
JP S58 88143 U describes a bent tubular container cylinder (6) integrally formed on one side of a base plate (5) having the same shape and the same size as the slide glass (3), and a bent hole (7) of the container cylinder (6) is made to penetrate to the opposite side of the base plate (5). A cell (1) formed by forming a recess (16) around an opening of the hole (7) of the substrate (5), fitting an annular packing (17), and protruding a part of the packing (17) from the substrate (5), and a bottom plate (9) having the same shape and the same size as the slide glass (3), with three edges of the bottom plate (9) as a Oritate. Forming a triangular hook (Oritate) (10a) protruding from an edge of each of two side walls close to one open side, the triangular hook (10a) (wing) having a hypotenuse at an upper side; A horizontal tubular spring receiver (11) is formed at the edge of the side Oritate on the other side, one end is elastically opened and inserted into both ends of the spring receiver (11), intermediate parts (18) and (18) are bent toward the bottom plate (9), and the other end is bent at the intermediate part.
JP H02 91944 U describes a centrifugal smear cell holder wherein folded edges are formed on both edges in the width direction of a bottom plate to which a slide glass can be freely attached, and projected upward from each folded edge in the vicinity of one end part of both folded edges. This cell holder for centrifugal smearing is obtained by forming a hook opening in the same lateral direction in order to laterally engage a holding spring, forming the hook in an approximately U-shape, and bending the intermediate part of the holding spring of which both end parts are pivotally supported in the vicinity of the other end part of the folded and erected edge in the direction of projecting the intermediate part toward a bottom plate.
JP S62 102145 U describes a filter paper for centrifugal smearing held between a substrate and a slide glass of a cell for centrifugal smearing formed by connecting a container cylinder to one side of the substrate and opening a hole continuous to the inside of the container cylinder to the other side of the substrate. The filter paper for centrifugal smearing is characterized in that a water-repellent liquid is impregnated into the inner peripheral edge part of a through-hole formed at a position matching the hole opened in the substrate of the cell for centrifugal smearing.

### Citation List

### Patent Literature

PTL 1: JP-A-11-044621
PTL 2: JP-T-2006-513413
JP S62 83951 U
JP S58 88143 U
JP H02 91944 U
JP S62 102145 U

### Non Patent Literature

NPL 1: "Thermo Scientific Shandon Thin-layer Cell Preparation System", the Internet <URL:http://www.med.osaka-cu.ac.jp/Central-lab/16F/kikisitu/cytospin catalog.pdf>
NPL 2: "Thermo Scientific Shandon Cytoclip Slide Clip, TPX Sample Chambers and Filter Cards Instructions for Use", the Internet <URL:http://tools.thermofisher.com/ content/sfs/manuals/D21681~.pdf>

### Summary of Invention

### Technical Problem

The conventional centrifugal smearing sample container holders as exemplified in PTL 1, PTL 2, NPL 1, and NPL 2 each are configured to be provided with: a base part in which both side surface sections on a back side are extended to form through holes, and to respectively form receiving sections, and the side surface sections on a front side are extended to respectively form locking sections curved in the same direction; and a holding part in which both sides of a handle section are bent to form arm sections, and both ends of the arm sections are bent to form end sections, in which the end sections that are back-to-back and direct outward are inserted into the receiving sections and are subjected to crushing. Further, a container containing a liquid sample, a packing, and slide glass are disposed on a bottom surface section of the base part, and the handle section is moved so as to be turned to lock the holding part in the locking sections, thereby holding the container, the packing, and the slide glass.

In the related art, the end sections are back-to-back and direct outward, in a state where an outward biased force is applied to the end sections, when an operator moves the handle section so as to turn, an external force applied to the holding part causes an inward force to act on the end sections, which easily generates a twist. When the holding part is locked in the locking sections to hold the container, the packing, and the slide glass, in order to prevent a gap among the sample container, the packing, and the slide glass from generating and the liquid sample from leaking outside, predetermined portions in the arm sections each need to abut on the sample container accommodated in the bottom surface section. However, in the related art, a twist is easily generated in the holding part when the holding part is locked in the locking sections, the holding part is twisted to make it difficult to lock the holding part in the locking sections, and the holding part is twisted so that when the predetermined portions of the arm sections abut on the sample container accommodated in the bottom surface section, the ways of abutting are different between the arm sections, which loses a balance. Accordingly, a gap is generated among the sample container, the packing, and the slide glass, so that the liquid sample has leaked outside. In addition, the end sections are inserted into the receiving sections and are thereafter subjected to crushing, so that a dedicated tool is necessary when the centrifugal smearing sample container holder is assembled, which results in the increased number of assembling steps and the increased product cost.

### Solution to Problem

The present invention has been accomplished under the circumstances, has a structure that prevents a twist of the holding part when the operator causes the holding part to be locked in the locking sections, and addresses an object of providing a centrifugal smearing sample container holder having a structure which enables improvement of operability and easy assembly.

As one aspect, a solution as described below solves the problem.

The present invention includes: A centrifugal smearing sample container holder (1) configured to accommodate a sample container (51), a packing (52), and a slide glass (53), and comprising:
A base part (2) including a bottom surface section (2a) configured to accommodate the sample container (51), the packing (52), and the slide glass (53) by being stacked, side surface sections (2b), (2c) formed on both sides of the bottom surface section (2a), locking sections (2e), (2f) respectively formed in the side surface sections (2b), (2c), a back surface section (2d) formed on a back side of the bottom surface section (2a), and a curl section (2g) formed in the back surface section (2d); and
a holding part (3) including a handle section (3a) that is disposed on a front side of the bottom surface section (2a) along a short direction of the base part (2), arm sections (3e), (3f) formed on both sides of the handle section (3a), and end sections (3b), (3c) obtained by the arm sections (3e), (3f) being respectively extended, and rotatably fitted into the curl section (2g), wherein
the arm sections (3e), (3f) are respectively disposed, in a state where the arm sections (3e), (3f) on a side of the handle section (3a) have been locked in the locking sections (2e), (2f), such that predetermined portions thereof abut on the sample container (51) accommodated in the bottom surface section (2a), and
the end sections (3b), (3c) are disposed facing each other, and a length of the handle section (3a) is shorter than an interval between the respective locking sections (2e), (2f), and
   the base part (2) is made of sheet metal, and
the curl section (2g) is curved toward a front side (side of the notch 2r) of the back surface section (2d).

With the configuration, the end sections are disposed inward and facing each other, and when the operator moves the handle section so as to turn in order to cause the arm sections on the side of the handle section to be locked in the locking sections, an inward force acts on the end sections to cause the curl section to be in a state where an inward biased force is applied thereto, so that it is possible to prevent a twist of the holding part. Therefore, the holding part is easily locked in the locking sections, when predetermined portions of the arm sections abut on the sample container accommodated in the bottom surface section, the ways of abutting are similar between the arm sections, which maintains a balance. Therefore, the sample container, the packing, and the slide glass are being excellently brought into close contact with one another, so that it is possible to prevent the liquid sample from leaking outside. In addition, the assembly can be attained with the simple work in which the end sections are only fitted into the curl section, so that no dedicated tool is necessary, and the product cost can be suppressed. Therefore, the operability is improved, and the easy assembly structure is attained.

In the centrifugal smearing sample container holder, the length of the handle section is shorter than the interval between the respective locking sections. With the configuration, a deviation of a position where the operator presses the handle section when operating can be prevented, and when predetermined portions of the arm sections abut on the sample container accommodated in the bottom surface section, the ways of abutting are similar between the arm sections, which maintains a balance.

The length of the curl section is preferably shorter than the interval between the respective locking sections. With the configuration, when the centrifugal smearing sample container holder is detached and attached from and to a device, the curl section is not caught in the device, so that the operability is excellent.

The end sections are preferably urged in directions to narrow an interval therebetween. With the configuration, in a series of operations in which the operator moves the handle section so as to turn to cause the holding part to be locked in the locking sections, and to hold the sample container, the packing, and the slide glass, it is possible to prevent the end sections from coming off from the curl section. In other words, such a structure is obtained that when the handle section is moved so as to be turned, the end sections mounted by extension of the arms on a side where the deformation amount becomes large by the rotary movement are difficult to come off from the curl section.

The base part is made of sheet metal, and the curl section is curved toward a front side of the back surface section. With the configuration, it is possible to enhance the rigidity by reducing the length of the arm sections, and downsize the base part.

The holding part is made of an elastic metal wire, and in the arm sections, preferably, elbow sections bent in a direction of the locking sections are respectively formed on the side of the handle section, and pressing sections bent in a direction of the bottom surface section are respectively formed on a side of the end sections. With the configuration, the pressing sections apply a biased force that presses the sample container, the packing, and the slide glass to the side of the bottom surface section, so that it is possible to hold the sample container, the packing, and the slide glass by being brought into close contact with one another. Note that, the operator can operate the handle section at a position distant to some extent from the base part due to the elbow sections, thereby obtaining the centrifugal smearing sample container holder excellent in the operability.

### Advantageous Effects of Invention

With the present invention, because the end sections are disposed inward and facing each other, when an operator moves the handle section so as to turn in order to cause the arm sections on a side of the handle section to be locked in the locking sections, an inward force acts on the end sections to cause a state where an inward biased force is applied to the curl section, so that it is possible to prevent a twist of the holding part. In addition, the assembly can be attained with the simple work in which the end sections each are only fitted into the curl section, so that no dedicated tool is necessary, and the product cost can be suppressed. Therefore, the centrifugal smearing sample container holder having a structure which enables improvement of operability and easy assembly is implemented.

### Brief Description of Drawings

Fig. 1 is a schematic view illustrating an example of a centrifugal smearing sample container holder according to an embodiment of the present invention, and is a perspective view seen from an obliquely upper side.
Fig. 2 is a plan view of the centrifugal smearing sample container holder according to the embodiment.
Fig. 3 is a bottom view of the centrifugal smearing sample container holder according to the embodiment.
Fig. 4 is a front view of the centrifugal smearing sample container holder according to the embodiment.
Fig. 5 is a back view of the centrifugal smearing sample container holder according to the embodiment.
Fig. 6 is a right side view of the centrifugal smearing sample container holder according to the embodiment.
Fig. 7 is a left side view of the centrifugal smearing sample container holder according to the embodiment.
Fig. 8 is a plan view of a base part included in the centrifugal smearing sample container holder according to the embodiment.
Fig. 9 is a plan view of a holding part included in the centrifugal smearing sample container holder according to the embodiment.
Fig. 10 is a schematic view illustrating a state where a sample container, a packing, and slide glass are disposed, in the centrifugal smearing sample container holder according to the embodiment, and is a perspective view seen from an obliquely upper side.
Fig. 11 is a schematic view illustrating a state where the sample container, the packing, and the slide glass are held, in the centrifugal smearing sample container holder according to the embodiment, and is a perspective view seen from an obliquely upper side.
Fig. 12 is a schematic view illustrating an example of a centrifugal smearing device on which the centrifugal smearing sample container holder according to the embodiment is mounted, and is a perspective view seen from an obliquely upper side.
Fig. 13 is a II-II line cross-sectional view in which an internal structure in Fig. 12 is omitted.

### Description of Embodiments

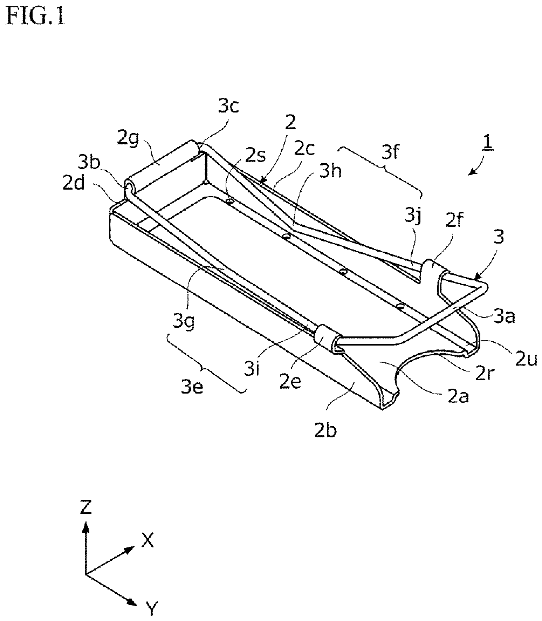

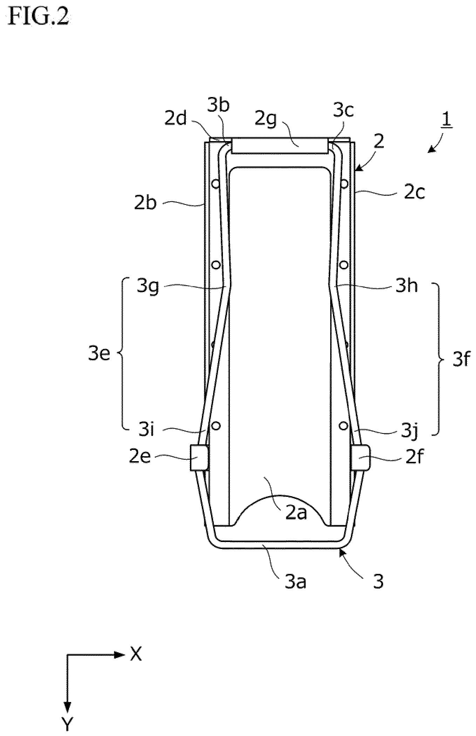

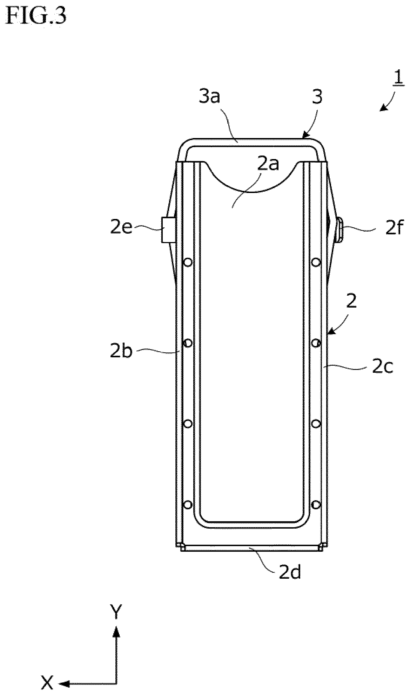

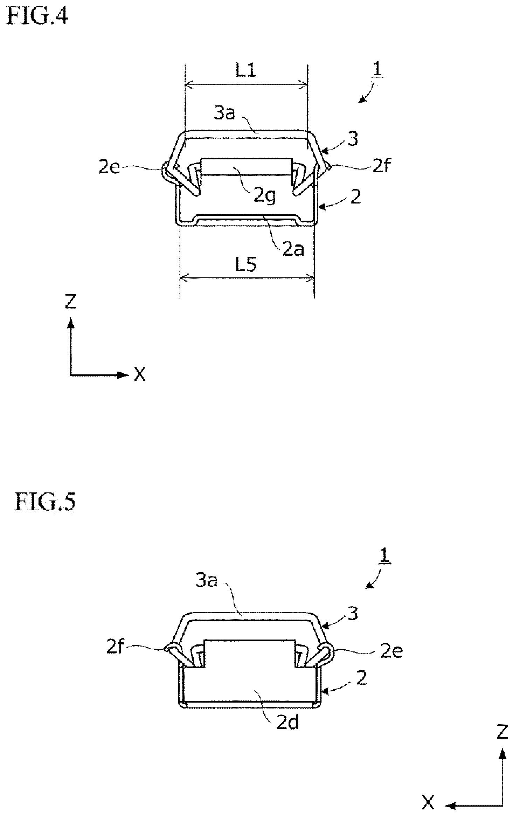

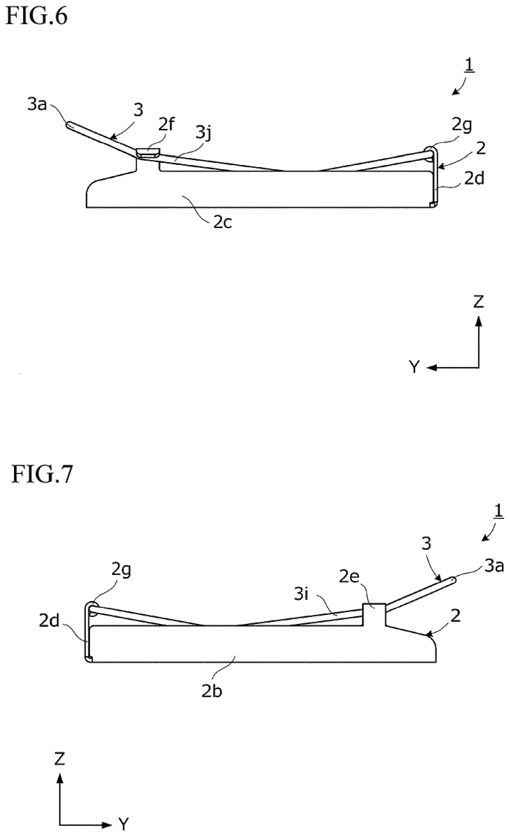

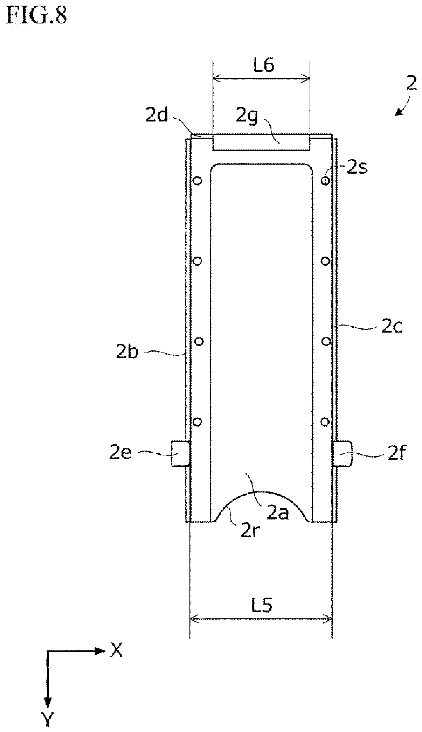

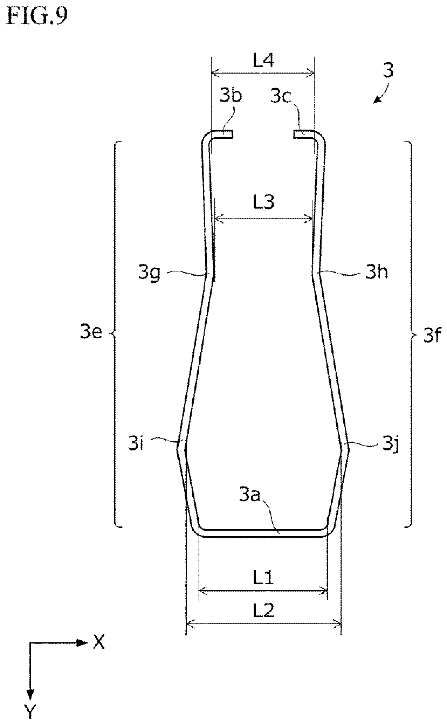

Hereinafter, an embodiment of the present invention will be described in detail with reference to the drawings. The present embodiment relates to, as one example, a centrifugal smearing sample container holder 1 that is attached to a centrifugal smearing device. Fig. 1 is a perspective view of the centrifugal smearing sample container holder 1 seen from an obliquely upper side. Fig. 2 is a plan view, Fig. 3 is a bottom view, Fig. 4 is a front view, Fig. 5 is a back view, Fig. 6 is a right side view, and Fig. 7 is a left side view, of the centrifugal smearing sample container holder 1. The centrifugal smearing sample container holder 1 includes a base part 2 and a holding part 3 by being combined with each other. Fig. 8 is a plan view of the base part 2. Fig. 9 is a plan view of the holding part 3. Note that, in all the drawings for explaining the embodiment, elements having the same function are assigned with the same reference numerals, and repeated explanations thereof are omitted in some cases.

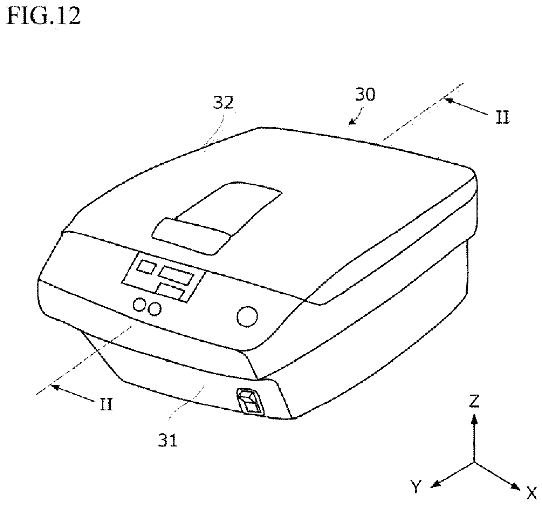

Fig. 12 is a schematic view illustrating an example of a centrifugal smearing device 30 on which the centrifugal smearing sample container holder 1 is mounted, and is a perspective view seen from an obliquely upper side. In the centrifugal smearing device 30, as one example, a lid part 32 is disposed on an upper side of a main body part 31, and an operation panel is in a front surface side of the main body part 31. The centrifugal smearing devices 30 can efficiently collect and smear cells for a short time using a centrifugal force, and are thus used in many pathology laboratories, research laboratories, and the like.

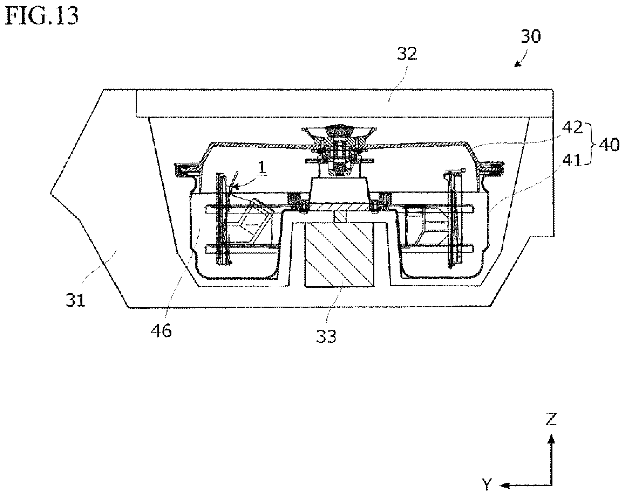

Fig. 13 is a II-II line cross-sectional view in which a part of an internal structure in Fig. 12 is omitted. The main body part 31 of the centrifugal smearing device 30 is provided with a motor 33 serving as a rotation driving unit, and a sealed rotating container 40, and the sealed rotating container 40 rotates by the rotation of the motor 33. The sealed rotating container 40 is provided with a storage part 41 capable of storing therein a plurality of the centrifugal smearing sample container holders 1, and a lid 42 that closes and seals a top surface opening section of the storage part 41. The centrifugal smearing sample container holder 1 is set to the storage part 41, in a state where a sample container 51, a packing 52, and slide glass 53 are held, at a position where a handle section 3a directs upward, such that a side of the sample container 51 is a side close to a rotation axis of the motor 33, and a side of the slide glass 53 is a side distant from the rotation axis of the motor 33.

Here, for an easy explanation of a positional relationship among respective sections of the centrifugal smearing sample container holder 1, directions are indicated by arrows of X, Y, and Z in the drawings. In the example illustrated in Fig. 13, when the centrifugal smearing sample container holder 1 is set to the centrifugal smearing device 30, the handle section 3a is directed upward. Note that, the arrangement configuration is not limited to this example, as long as smearing is possible by a centrifugal force.

In the sample container 51, a funnel-shaped accommodation part for accommodating a liquid sample to be used in a cytological diagnosis or the like is formed, and a prescribed passage directed outward is formed on a side surface of a bottom section of the accommodation part. When the centrifugal smearing sample container holder 1 is set to the centrifugal smearing device 30 and is rotated, and a centrifugal force is applied thereto, the liquid sample passes through the prescribed passage and moves outward. An opening part is formed outward of the prescribed passage, and the slide glass 53 is disposed outward of the opening part. The packing 52 is disposed between the opening part and the slide glass 53 so as to seal a portion between the opening part and the slide glass 53. When filter paper with openings serving as the packing 52 is disposed, cells are smeared on the slide glass 53, and the liquid component is oozed into the filter paper, so that it is possible to collect and smear the limited floating cells in the liquid sample with high efficiency. The filter paper allows the cytoplasm to extend, and is thus advantageous for the identification of a cell, and is difficult to exfoliate. Moreover, the filter paper is used for drying and fixing. When a rubber plate with openings serving as the packing 52 is disposed, cells are smeared on the slide glass 53, the liquid component is difficult to scatter outward from the rubber plate, and can be repeatedly used by being cleaned. The rubber plate is used for a sample having a small number of floating cells and the like. The type of the capacity of the sample container 51 is, for example, 1 [mL], 6 [mL], and 12 [mL]. The filter paper or the rubber plate is used as appropriate as the packing 52 depending on the type and the amount of the liquid sample. As for the sample container 51, the packing 52, and the slide glass 53, known ones can be used.

The centrifugal smearing sample container holder 1 includes the base part 2 illustrated in Fig. 8 and the holding part 3 illustrated in Fig. 9 by being combined with each other.

The base part 2 can be produced by sheet-metal working, casting, cutting, laser beam machining, etching, die molding, and other known processing methods. The base part 2 is made of sheet metal to allow easy production, and weight reduction while enhancing the rigidity. As for materials of the base part 2, for example, hard metal such as iron, stainless steel, aluminum, a nickel alloy, and a titanium alloy, chemical-resistant resins such as polyimide (PI), polyether ether ketone (PEEK), polyamideimide (PAI), polybenzoimidazole (PBI), polyphenylenesulfide (PPS), polytetrafluoroethylene (PFA), and a liquid crystal polymer, ceramic, and other known structural materials are applicable.

In the present embodiment, the base part 2 is formed by sheet metal such as stainless steel by being subjected to press processing or bending, and includes: a bottom surface section 2a to which the sample container 51, the packing 52, and the slide glass 53 are disposed; a side surface section 2b formed on a left side of the bottom surface section 2a, and a side surface section 2c formed on a right side of the bottom surface section 2a, in plan view; a locking section 2e formed on the side surface section 2b, and a locking section 2f formed on the side surface section 2c; a back surface section 2d formed on a back side of the bottom surface section 2a; and a curl section 2g formed on the back surface section 2d. Through holes 2s are formed on each of the left and right sides of the bottom surface section 2a at predetermined intervals. Cells are smeared on the slide glass 53 through the through holes 2s by a centrifugal force, and the moisture having oozed out from the filter paper by the centrifugal force is quickly scattered from the through holes 2s via a groove 2u formed on the bottom surface section 2a. Moreover, the through holes 2s facilitate liquid draining and drying in cleaning. A notch 2r having a circular arc shape is formed in the center of the bottom surface section 2a on the front side. The notch 2r enables the slide glass 53 to be easily disposed and taken out without a fingertip being caught in the bottom surface section 2a.

The holding part 3 has spring characteristics, and can be produced by bending, sheet-metal working, casting, cutting, laser beam machining, etching, and other known processing methods, for an elastic metal wire. The holding part 3 is made of an elastic metal wire by being subjected to bending accordingly to have a structure of easy production, thereby allowing enhancement of the rigidity and weight reduction while having spring characteristics. As for materials of the holding part, for example, high elastic metal such as iron, stainless steel, a nickel alloy, and a titanium alloy, resin molding, ceramic, and other known spring materials are applicable.

The holding part 3 is formed by an elastic metal wire such as stainless steel by being subjected to bending, and includes the handle section 3a, an arm section 3e formed on a left side of the handle section 3a, and an arm section 3f formed on a right side of the handle section 3a, in plan view. The arm section 3e is extended and an end portion thereof is bent at an approximate right angle to form an end section 3b, and the arm section 3f is extended and an end portion thereof is bent at an approximate right angle to form an end section 3c.

Further, in the arm section 3e, an elbow section 3i that is bent at an obtuse angle in a direction of the locking section 2e is formed on a side of the handle section 3a, and in the arm section 3f, an elbow section 3j that is bent at an obtuse angle in a direction of the locking section 2f is formed on the side of the handle section 3a. In addition, in the arm section 3e, a pressing section 3g that is bent at an obtuse angle in a direction of the bottom surface section 2a is formed on a side of the end section 3b, and in the arm section 3f, a pressing section 3h that is bent at an obtuse angle in a direction of the bottom surface section 2a is formed on a side of the end section 3c.

Here, the arm section 3e and the arm section 3f; the elbow section 3i and the elbow section 3j; the pressing section 3g and the pressing section 3h; and the end section 3b and the end section 3c are respectively disposed to be linearly symmetrical. A wire diameter of the wire is set, for example, 1.0 [mm] or more and 2.0 [mm] or less. As the wire diameter of the wire becomes larger, the rigidity becomes larger, and as the wire diameter of the wire becomes smaller, the elastic property becomes larger. The wire diameter of the wire is set as appropriate depending on the materials, the sizes, and the like of the sample container 51, the packing 52, and the slide glass 53. The pressing section 3g and the pressing section 3h are not limited to the simple bent shape, but may be a winding shape of one to two rounds.

As illustrated in Fig. 1 to Fig. 7, in the centrifugal smearing sample container holder 1, the end section 3b and the end section 3c are rotatably fitted into the curl section 2g, respectively, and the handle section 3a is thus rotatably disposed with respect to the base part 2.

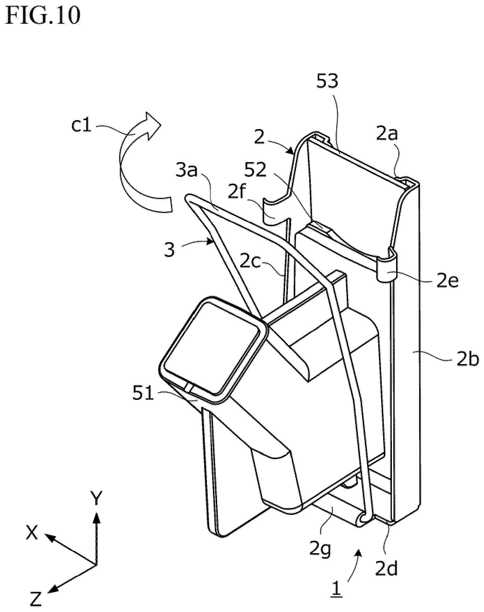

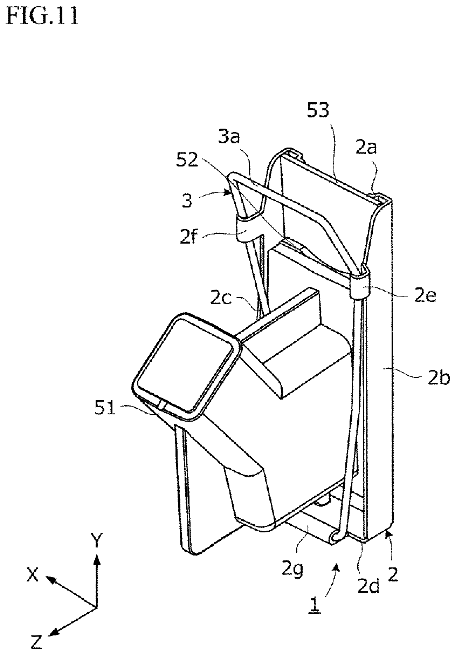

Further, when the sample container 51, the packing 52, and the slide glass 53 are held, as illustrated in Fig. 10 and Fig. 11, the sample container 51, the packing 52, and the slide glass 53 are disposed on the bottom surface section 2a by being stacked. Next, the handle section 3a is moved so as to be turned in a direction of an arrow c1, the elbow section 3i is locked in the locking section 2e, and the elbow section 3j is locked in the locking section 2f. Further, the pressing section 3g and the pressing section 3h abut on and press the sample container 51 against the side of the bottom surface section 2a, thereby holding the sample container 51, the packing 52, and the slide glass 53 so as to be brought into close contact with one another.

With the present invention, in the holding part 3, the end section 3b and the end section 3c are disposed facing each other. With the configuration, an end surface of the end section 3b and an end surface of the end section 3c face each other and direct inward, so that the curl section 2g becomes a state in which an inward biased force is applied thereto. Here, in order to lock the elbow section 3i in the locking section 2e, and to lock the elbow section 3j in the locking section 2f, when an operator moves the handle section 3a so as to turn in the direction of the arrow c1, a twist of the holding part 3 is prevented, and when the operator causes the elbow section 3i to be locked in the locking section 2e and the elbow section 3j to be locked in the locking section 2f, a warp of the holding part 3 is prevented. Further, in the both arm section 3e and 3f, in a state where the elbow section 3i is locked in the locking section 2e and the elbow section 3j is locked in the locking section 2f, the pressing section 3g and the pressing section 3h abut on and press the sample container 51 against the side of the bottom surface section 2a, so that in a series of works, the sample container 51, the packing 52, and the slide glass 53 are brought into close contact with one another. This attains the structure that prevents the liquid sample from leaking, thereby enhancing the reliability.

In addition, with the configuration, the assembly can be attained with the simple work in which the end section 3b and the end section 3c are only fitted into the curl section 2g, respectively, so that no dedicated tool is necessary, thereby obtaining the configuration that attains the easy assembly in a pathology laboratory, a research laboratory, or the like. For example, the base part 2 and the holding part 3 can be individually purchased, and the maintenance cost can be suppressed.

The present embodiment has such a configuration that, in a state where the holding part 3 and the base part 2 are separated from each other, an interval L4 between the end section 3b and the end section 3c is shorter than a length L6 of the curl section 2g (see Fig. 9), and in a state where each of the end section 3b and the end section 3c is rotatably fitted into the curl section 2g, the holding part 3 urges a force in a direction to narrow the interval L4. With the configuration, in a series of operations in which the handle section 3a is moved so as to be turned in the direction of the arrow c1 to lock the arm section 3e in the locking section 2e and to lock the arm section 3f in the locking section 2f, so that the sample container 51, the packing 52, and the slide glass 53 are brought into close contact with one another and held, a state where each of the end section 3b and the end section 3c is fitted into the curl section 2g is maintained. In other words, it is possible to prevent the end section 3b and the end section 3c from coming off from the curl section 2g.

The present invention has such a configuration that a length L1 of the handle section 3 a is shorter than an inner interval L5 between the locking section 2e and the locking section 2f (see Fig. 4). With the configuration, it is possible to prevent the deviation of a position at which the operator presses the handle section 3a when operating.

Further, the length L6 of the curl section 2g is shorter than the inner interval L5 between the locking section 2e and the locking section 2f. With the configuration, in a state where the respective arm sections 3e, 3f of the holding part 3 are respectively locked in the respective locking sections 2e, 2f, when the sample container 51, the packing 52, and the slide glass 53 are held, a biased force that presses them to the side of the bottom surface section 2a is applied, so that it is possible to hold the sample container 51, the packing 52, and the slide glass 53 by enhancing the adhesiveness among them.

The locking section 2e and the locking section 2f are curved in the same direction, and both are formed at positions outward of the bottom surface section 2a. With the configuration, when the sample container 51, the packing 52, and the slide glass 53 are disposed on the bottom surface section 2a, it is possible to prevent the sample container 51, the packing 52, and the slide glass 53 from abutting on the locking section 2e and the locking section 2f. In addition to this, the structure in which the handle section 3a is moved so as to be turned in the direction of the arrow c1 to easily lock the respective arm sections 3e, 3f of the holding part 3 in the respective locking sections 2e, 2f is attained.

The curl section 2g is curved so as to locate at a position on the front side (side of the notch 2r) of the back surface section 2d. With the configuration, it is possible to enhance the rigidity by making the lengths of the arm sections 3e, 3f short, respectively, and downsize the base part 2.

In the present embodiment, at the positions where the arm sections 3e, 3f are respectively locked in the locking sections 2e, 2f, the pressing sections 3g, 3h bent at an obtuse angle in the direction of the bottom surface section 2a are respectively formed in the arm sections 3e, 3f. With the configuration, the pressing sections 3g, 3h apply a biased force that presses the sample container 51, the packing 52, and the slide glass 53 to the side of the bottom surface section 2a, so that it is possible to hold the sample container 51, the packing 52, and the slide glass 53 by being brought into close contact with one another. Further, in the arm sections 3e, 3f, at the side of the handle section 3a, the elbow section 3i bent in the direction of the locking section 2e and the elbow section 3j bent in the direction of the locking section 2f are respectively formed. With the configuration, the operator can operate the handle section 3a at a position distant to some extent from the base part 2, thereby obtaining the centrifugal smearing sample container holder 1 excellent in the operability.

The present invention is not limited to the embodiment having been described above, but various changes can be made without deviating from the scope of the present invention. For example, in the above mentioned embodiment, the configuration in which the base part 2 is formed by the sheet metal being subjected to press processing or bending has been exemplified, but is not limited thereto, and the base part 2 can be produced by casting, cutting, laser beam machining, etching, and other known processing methods. Moreover, in the above mentioned embodiment, the configuration in which the holding part 3 is formed by an elastic metal wire being subjected to bending has been exemplified, but is not limited thereto, as long as the holding part 3 has spring characteristics, the holding part 3 can be produced by resin mold molding, sheet metal, casting, cutting, laser beam machining, etching, and other known processing methods.

## Claims

1. A centrifugal smearing sample container holder (1) configured to accommodate a sample container (51), a packing (52), and a slide glass (53), and comprising:
A base part (2) including a bottom surface section (2a) configured to accommodate the sample container (51), the packing (52), and the slide glass (53) by being stacked, side surface sections (2b), (2c) formed on both sides of the bottom surface section (2a), locking sections (2e), (2f) respectively formed in the side surface sections (2b), (2c), a back surface section (2d) formed on a back side of the bottom surface section (2a), and a curl section (2g) formed in the back surface section (2d); and
a holding part (3) including a handle section (3a) that is disposed on a front side of the bottom surface section (2a) along a short direction of the base part (2), arm sections (3e), (3f) formed on both sides of the handle section (3a), and end sections (3b), (3c) obtained by the arm sections (3e), (3f) being respectively extended, and rotatably fitted into the curl section (2g), wherein
the arm sections (3e), (3f) are respectively disposed, in a state where the arm sections (3e), (3f) on a side of the handle section (3a) have been locked in the locking sections (2e), (2f), such that predetermined portions thereof abut on the sample container (51) accommodated in the bottom surface section (2a), and
the end sections (3b), (3c) are disposed facing each other, and
a length of the handle section (3a) is shorter than an interval between the respective locking sections (2e), (2f), and
the base part (2) is made of sheet metal, and
the curl section (2g) is curved toward a front side of the back surface section (2d).

2. The centrifugal smearing sample container holder (1) according to claim wherein a length of the curl section (2g) is shorter than the interval between the respective locking sections (2e), (2f), and
The locking sections (2e), (2f) are curved in the same direction, and both are formed at positions outward of the bottom surface section (2a).

3. The centrifugal smearing sample container holder (1) according to claim 1 or 2, wherein the end sections (3b), (3c) are urged in directions to narrow an interval therebetween.

4. The centrifugal smearing sample container holder (1) according to any one of claims 1 to 3, wherein
The holding part (3) is made of an elastic metal wire, and
the diameter of the elastic metal wire is 1.0 mm or more and 2.0 mm or less, and
in the arm sections (3e), (3f), elbow sections (3i), (3j) bent in a direction of the locking sections (2e), (2f) are respectively formed at the side of the handle section (3a), and pressing sections (3g), (3h) bent in a direction of the bottom surface section (2a) are respectively formed on a side of the end sections (3b), (3c), and
the arm sections (3e), (3f); the elbow sections (3i), (3j); the pressing sections (3g), (3h); and the end sections (3b), (3c) are respectively disposed to be linearly symmetrical.

## Patentansprüche

1. Zentrifugalabstrichprobenbehälterhalterung (1), die dazu ausgelegt ist, einen Probenbehälter (51), eine Packung (52) und einen Objektträger (53) aufzunehmen, und Folgendes umfasst:
einen Basisteil (2), umfassend einen Bodenflächenabschnitt (2a), der dazu ausgelegt ist, den Probenbehälter (51), die Packung (52) und den Objektträger (53) durch Stapeln aufzunehmen, Seitenflächenabschnitte (2b), (2c), die auf beiden Seiten des Bodenflächenabschnitts (2a) ausgebildet sind, Verriegelungsabschnitte (2e), (2f), die jeweils in den Seitenflächenabschnitten (2b), (2c) ausgebildet sind, einen Rückflächenabschnitt (2d), der auf einer Rückseite des Bodenflächenabschnitts (2a) ausgebildet ist, und einen Einrollabschnitt (2g), der im Rückflächenabschnitt (2d) ausgebildet ist; und
einen Halteteil (3), umfassend einen Griffabschnitt (3a), der auf einer Vorderseite des Bodenflächenabschnitts (2a) entlang einer kurzen Richtung des Basisteils (2) angeordnet ist, Armabschnitte (3e), (3f), die auf beiden Seiten des Griffabschnitts (3a) ausgebildet sind, und Endabschnitte (3b), (3c), die durch die Armabschnitte (3e), (3f) erhalten werden, indem diese jeweils erweitert und rotierbar in den Einrollabschnitt (2g) eingepasst werden, wobei
die Armabschnitte (3e), (3f) in einem Zustand, in dem die Armabschnitte (3e, 3f) auf einer Seite des Griffabschnitts (3a) in den Verriegelungsabschnitten (2e), (2f) verriegelt wurden, jeweils so angeordnet sind, dass vorbestimmte Abschnitte davon an den Probenbehälter (51) angrenzen, der im Bodenflächenabschnitt (2a) aufgenommen ist, und
die Endabschnitte (3b), (3c) einander zugewandt angeordnet sind und
eine Länge des Griffabschnitts (3a) kürzer ist als ein Abstand zwischen den entsprechenden Verriegelungsabschnitten (2e), (2f) und
der Basisteil (2) aus Metallblech ausgebildet ist und
der Einrollabschnitt (2g) in Richtung einer Vorderseite des Rückflächenabschnitts (2d) gekrümmt ist.

2. Zentrifugalabstrichprobenbehälterhalterung (1), nach Anspruch 1, wobei eine Länge des Windungsabschnitts (2g) kürzer ist als der Abstand zwischen den entsprechenden Verriegelungsabschnitten (2e), (2f) und wobei
die Verriegelungsabschnitte (2e), (2f) in die gleiche Richtung gekrümmt sind und beide in Positionen außerhalb des Bodenflächenabschnitts (2a) ausgebildet sind.

3. Zentrifugalabstrichprobenbehälterhalterung (1) nach Anspruch 1 oder 2, wobei die Endabschnitte (3b), (3c) in Richtungen gedrückt werden, um einen Abstand zwischen diesen zu verkleinern.

4. Zentrifugalabstrichprobenbehälterhalterung (1) nach einem der Ansprüche 1 bis 3, wobei
der Halteteil (3) aus einem elastischen Metalldraht besteht und
der Durchmesser des elastischen Metalldrahts 1,0 mm oder mehr und 2,0 mm oder weniger beträgt und
in den Armabschnitten (3e), (3f) sind Ellbogenabschnitte (3i), (3j), die in Richtung der Verriegelungsabschnitte (2e), (2f) gebogen sind, jeweils auf der Seite des Griffabschnitts (3a) ausgebildet und Druckabschnitte (3g), (3h), die in Richtung des Bodenflächenabschnitts (2a) gebogen sind, jeweils auf einer Seite der Endabschnitte (3b), (3c) ausgebildet, und
die Armabschnitte (3e), (3f), die Ellbogenabschnitte (3i), (3j), die Druckabschnitte (3g), (3h) und die Endabschnitte (3b), (3c) sind jeweils angeordnet, um linear symmetrisch zu sein.

## Revendications

1. Support de récipient d'échantillon de frottis centrifuge (1) configuré pour recevoir un récipient d'échantillon (51), une garniture (52) et un verre coulissant (53), et comprenant :
une partie de base (2) incluant une section de surface inférieure (2a) configurée pour loger le récipient d'échantillon (51), la garniture (52) et le verre coulissant (53) en étant empilés, des sections de surface latérales (2b), (2c) formées sur les deux côtés de la section de surface inférieure (2a), des sections de verrouillage (2e), (2f) formées respectivement dans les sections de surface latérales (2b), (2c), une section de surface arrière (2d) formée sur un côté arrière de la section de surface inférieure (2a) et une section de boucle (2g) formée dans la section de surface arrière (2d) ; et
une partie de maintien (3) incluant une section de poignée (3a) qui est disposée sur un côté avant de la section de surface inférieure (2a) le long de la direction courte de la partie de base (2), des sections de bras (3e), (3f) formées sur les deux côtés de la section de poignée (3a), et des sections d'extrémité (3b), (3c) obtenues par les sections de bras (3e), (3f) étant respectivement étendues et ajustées de manière rotative dans la section de boucle (2g), dans lequel
les sections de bras (3e), (3f) sont respectivement disposées, dans un état dans lequel les sections de bras (3e), (3f) sur un côté de la section de poignée (3a) ont été verrouillées dans les sections de verrouillage (2e), (2f), de telle sorte que des parties prédéterminées de celles-ci viennent buter sur le récipient d'échantillon (51) logé dans la section de surface inférieure (2a), et
les sections d'extrémité (3b), (3c) sont disposées l'une en face de l'autre, et
une longueur de la section de poignée (3a) est inférieure à un intervalle entre les sections de verrouillage respectives (2e), (2f), et
la partie de base (2) est réalisée en tôle, et
la section de boucle (2g) est incurvée vers un côté avant de la section de surface arrière (2d).

2. Support de récipient d'échantillon de frottis centrifuge (1) selon la revendication 1, dans lequel :
une longueur de la section de boucle (2g) est inférieure à l'intervalle entre les sections de verrouillage respectives (2e), (2f), et
les sections de verrouillage (2e), (2f) sont incurvées dans la même direction, et les deux sont formées dans des positions vers l'extérieur de la section de surface inférieure (2a).

3. Support de récipient d'échantillon de frottis centrifuge (1) selon la revendication 1 ou 2, dans lequel les sections d'extrémité (3b), (3c) sont poussées dans des directions pour réduire un intervalle entre celles-ci.

4. Support de récipient d'échantillon de frottis centrifuge (1) selon l'une quelconque des revendications 1 à 3, dans lequel
la partie de maintien (3) est réalisée en un fil métallique conducteur, et
le diamètre du fil métallique élastique est de 1,0 mm ou plus et de 2,0 mm ou moins, et
dans les sections de bras (3e), (3f), des sections de coude (3i), (3j) pliées dans la direction des sections de verrouillage (2e), (2f) sont formées respectivement sur le côté de la section de poignée (3a), et des sections de pression (3g), (3h) pliées dans la direction de la section de surface inférieure (2a) sont formées respectivement sur un côté des sections d'extrémité (3b), (3c), et
les sections de bras (3e), (3f) ; les sections de coude (3i), (3j) ; les sections de pression (3g), (3h) ; et les sections d'extrémité (3b), (3c) sont respectivement disposées pour être linéairement symétriques.
